# EUROPEAN PATENT SPECIFICATION

(11) **EP 2 303 265 B1**
(45) Date of publication and mention of the grant of the patent: **10.12.2014**
(21) Application number: 09838708.7
(22) Date of filing: 03.06.2009
(51) Int. Cl.: A61K 31/4196, A61K 31/506, A61K 47/44, A61P 31/10

(54) **STABLE TOPICAL FORMULATION COMPRISING VORICONAZOLE**
STABILE TOPISCHE FORMULIERUNG MIT VORICONAZOL
FORMULATION TOPIQUE STABLE COMPRENANT DU VORICONAZOLE

(30) Priority: 06.06.2008 IN MU12042008; 24.07.2008 IN MU15872008; 16.09.2008 IN MU19672008; 07.10.2008 US 103315 P
(43) Date of publication of application: 06.04.2011
(73) Proprietor: Glenmark Pharmaceuticals Limited, Mumbai 400 099 (IN)
(72) Inventor: DHUPPAD, Ulhas, Rameshchandra, Nashik 422 101 Maharashtra (IN); KHACHANE, Vasant, Sitaram, Maharashtra (IN); BHAMRE, Nitin, Babulal, Maharashtra (IN); SATPUTE, Ravindra, Moreshwar, Nashik 422 011 Maharashtra (IN); MAHAJAN, Narayan, Tukaram, NASIK-42213, Maharashtra (IN); BASA, Shradhanjali, Mayurbhanj 757 001 Orissa (IN); KOTWAL, Rupesh, Subhashchandra, Nashik-Pune Road Nashik 422 011 (IN); SOMNATHE, Nitin, Dashrathrao, Wardha 442 104 Maharashtra (IN)
(74) Representative: Atkinson, Jonathan David Mark
(86) International application number: PCT/IN2009/000316
(87) International publication number: WO 2010/084505

(56) References cited:
- EP-A2- 0 982 031
- WO-A1-2008/045186
- WO-A2-2007/012967
- US-A- 5 773 443
- US-A1- 2004 209 852
- US-A1- 2007 196 452
- US-A1- 2007 286 812
- US-A1- 2008 063 607

## Description

The present invention relates to a stable topical formulation comprising voriconazole or its pharmaceutically acceptable salt; and a process for preparing the same. Particularly, the present invention relates to a stable topical formulation comprising an effective amount of voriconazole or its pharmaceutically acceptable salt, optionally a corticosteroid and a pharmaceutical carrier; and use of such formulation for the treatment of local or non-systemic fungal infections in a subject.

### BACKGROUND

Voriconazole (Formula I) is designated chemically as (2R,3S)-2-(2,4-difluorophenyl)-3-(5-fluoro-4-pyrimidinyl)-1-(1H-1,2,4-triazol-1-yl)-2-butanol with an empirical formula of C₁₆H₁₄F₃N₅O and a molecular weight of 349.3.

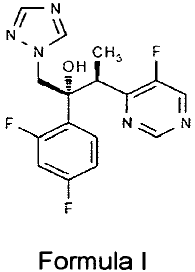

Voriconazole is commercially available as a lyophilized powder for preparing a solution for intravenous infusion, as a film-coated tablet for oral administration, and also as a powder for preparing an oral suspension (VFEND^{®}, marketed by Pfizer). Voriconazole is indicated for the treatment of various fungal infections caused by *Aspergillus fumigatus* and *Aspergillus* other than *A*. *fumigatus,* Candidemia, Esophageal candidiasis and serious fungal infections caused by *Scedosporium apiospermum.*

Voriconazole is disclosed in European Patent Application EP 0440372. U.S. Patent Nos. 5,116,844; 5,364,938; 5,567,817; 5,773,443 and 6,632,803 describe voriconazole and its formulations.

U.S. Patent Application Publication No. 2005/0043251 relates to a composition comprising various antifungal agents for the topical treatment of otitis externa. PCT Patent Application Publication No. WO 2005/051353 relates to an aqueous poloxamer-micelle based formulation comprising voriconazole for parenteral administration.

PCT Patent Application Publication No. WO2008045186 relates to a composition for treatment of otitis externa with topical and orally administered antifungal agents including voriconazole.

PCT Patent Application Publication No. WO2007012967 relates to a combination therapy including voriconazole and fluconazole.

European Patent Application Publication No. EP0982031 relates to a combination therapy including voriconazole and alatrofloxacin.

### SUMMARY

The present invention relates to a stable topical formulation in the form of a cream comprising from 0.01% w/w to 5% w/w voriconazole or its pharmaceutically acceptable salt and a pharmaceutical carrier, wherein said formulation contains not more than 5% w/w of water, and not more than 5.5% w/w 2,4-difluoro-2-(1H)-1,2,4-triazol-1-ylacetophenone upon storage at a temperature of about 40°C and a relative humidity of about 75% for a period of 3 months.

In an embodiment, the stable topical formulation comprising an effective amount of voriconazole or its pharmaceutically acceptable salt that ranges from about 0.01% to about 5% w/w, or preferably from about 0.05% to about 2% w/w (based on total weight of the formulation).

In another embodiment, the stable topical formulation is substantially free of water. Preferably, the stable topical formulation of the present invention contains not more than about 3%, or not more than about 2% w/w of water (based on total weight of the formulation). Alternatively, the stable topical formulation of the present invention can be anhydrous (free of water).

In yet another embodiment, the topical formulation of present invention possesses storage stability at accelerated conditions, i.e., the formulation contains not more than 9% w/w total impurities (based on total weight of the formulation) formed upon storage at a temperature of about 40 °C and a relative humidity of about 75% for a period of 3 months. The formulation contains not more than 5.5% w/w of 2,4-difluoro-2-(1H)-1,2,4-triazol-I-ylacetophenone (an impurity generated upon hydrolysis of voriconazole, hereinafter designated as impurity A), formed upon similar storage for a period of 3 months (based on total weight of the formulation).

In a specific embodiment of present invention, the stable topical formulation comprising voriconazole or its pharmaceutically acceptable salt is a cream formulation substantially free of water, wherein the cream formulation contains not more than 9% w/w total impurities (based on total weight of the formulation) formed upon storage at a temperature of about 40 °C and relative humidity of about 75% for a period of at least 3 months. The cream formulation contains not more than 5.5% w/w impurity A (based on total weight of the formulation) formed upon similar storage for a period of 3 months.

Another specific embodiment of present invention is a stable topical formulation comprising an effective amount of voriconazole or its pharmaceutically acceptable salt (as a first active ingredient), and a corticosteroid (as a second active ingredient), and a pharmaceutical carrier, wherein said topical formulation is substantially free of water. Preferably, the corticosteroid includes clobetasol, halobetasol, fluocinonide, betamethasone, dexamethasone, beclomethasone, alcomethasone, diflorasone, fluticasone, hydrocortisone, mometasone, fluocinolone, desonide, and triamcinolone.

In the context of present invention, for topical use, the effective amount of the corticosteroid ranges from about 0.005% to about 5% w/w, or preferably from about 0.01% to about 3% w/w (based on total weight of the formulation). Preferred weight percentage ranges (based on total weight of the formulation) for various corticosteroids that are contemplated herein include: mometasone (in the range of 0.01% to 1%), betamethasone (in the range of 0.01 to 1%), fluocinolone (in the range of 0.01 to 1%), beclomethasone (in the range of 0.01 to 1 %), desonide (in the range of 0.01 to 1 %), fluticasone (in the range of 0.01 to 1%), hydrocortisone (in the range of 0.01 to 5%), dexamethasone (in the range of 0.01 to 1 %), alcomethasone (in the range of 0.01 to 1 %), and diflorasone (in the range of 0.01 to 1 %).

More preferably, the stable topical formulation of the present invention includes:
a) voriconazole in the range of about 0.05% to about 2% w/w;
b) mometasone furoate in the range of about 0.01% to about 1% w/w; and
c) a pharmaceutical carrier,
wherein said formulation is substantially free of water.

A further embodiment is a process for preparing a stable topical formulation comprising voriconazole or its pharmaceutically acceptable salt and a pharmaceutical carrier, comprising (a) co-melting one or more excipients by heating them up to a temperature of about 75 °C to form a molten mass; (b) obtaining a dispersion of voriconazole or a salt thereof in a solvent; (c) mixing the dispersion obtained in step (b) in the molten mass under stirring; and (d) homogenizing the mixture for about 15-30 min and cooling it gradually to ambient condition.

Also disclosed is a method for treating a local or non-systemic fungal infection in a subject in need thereof, said method comprising applying to an afflicted region of the subject a stable topical formulation comprising an effective amount of voriconazole or its pharmaceutically acceptable salt. Preferably, the subject includes mammal such as human.

### BRIEF DESCRIPTION OF THE DRAWINGS

Figure 1: Bar graph depicting the levels of Impurity A and Total impurities when voriconazole 0.5% w/w cream formulations with varying amounts of water were stored at about 40 °C temperature and a relative humidity of about 75 % for a period of 3 months.

### DETAILED DESCRIPTION

The definitions of the terms used herein follow. However, where a definition set forth in the present application relative to one in an earlier provisional application (from which the priority is claimed) are in conflict, the definition in the present application shall control the meaning of the term(s).

Voriconazole is unstable in water (undergoes hydrolysis, forms an inactive enantiomer from the recombination of the retro-aldol products). This instability in water leads to increasing levels of impurities over time. High levels of impurities in turn results in formulations that have reduced or otherwise impaired activity. Thus, the shelf life of topical voriconazole formulations containing water is greatly reduced in comparison to other topical formulations. The development, on a commercial scale, of a stable topical formulation of voriconazole poses a challenge, which heretofore, has yet been unmet. The present invention, surprisingly, provides the answer with a stable topical formulation comprising voriconazole or its pharmaceutically acceptable salt, wherein said topical formulation possesses the storage stability at accelerated conditions (i.e., a temperature of about 40 °C and a relative humidity of about 75%) for a period of at least 3 months.

Without being bound by any particular theory, it would appear by the data gathered and presented herein that the water content relative to voriconazole or its pharmaceutically acceptable salt, prepared as herein described, leads to a stable topical formulation. Such stable topical formulation of voriconazole or its pharmaceutically acceptable salt, prepared as herein described, can be used for the treatment of local or non-systemic fungal infections and seborrheic dermatitis in subjects, in need thereof.

Further, a combination therapy for the treatment of co-existing fungal diseases and seborrheic dermatitis is advisable, particularly when two or more active ingredients have different modes of action. For example, a composition comprising voriconazole as a first active ingredient and a corticosteroid as a second active ingredient, as discovered by the inventors of the present invention, can be very effective in treating local or non-systemic fungal diseases and seborrheic dermatitis in a subject.

The present invention relates to a stable topical formulation comprising an effective amount of voriconazole or its pharmaceutically acceptable salt and a pharmaceutical carrier.

The term "topical formulation" (synonymously, "topical composition") is used herein to refer to a pharmaceutical preparation intended for topical or local application to an afflicted region of a subject in need thereof, and includes such dosage forms as gel, cream, ointment, emulsion, suspension, solution, drops, lotion, paint, pessary, douche, suppository, troche, spray, sponge, film, or foam. Preferably, the topical formulation is in the form of a cream, or an ointment. The present invention contemplates certain types of the topical formulations that include shampoo preparations; preparations for nail (like lacquers, paints, varnishes, top coats, base coats, nail hardeners and ridge fillers); vaginal and rectal formulations (like tablet, tampon, ovule, soft gelatin capsule, and ring); and mouth paints.

As used herein, the terms "effective amount" or "topically effective amount" of a voriconazole or its pharmaceutically acceptable salt refers to a non-toxic but sufficient amount of the active ingredient to provide the desired effect when administered topically. The "effective amount" will vary depending on the disease and its severity and the age, weight, physical condition and responsiveness of the subject to be treated.

In an embodiment, the stable topical formulation comprises an effective amount of voriconazole or its pharmaceutically acceptable salt that ranges from about 0.01% to about 5% w/w, or preferably from about 0.05% to about 2% w/w (based on total weight of the formulation). More preferably, the stable topical formulation of the present invention comprises about 0.1% to about 2% w/w voriconazole or its pharmaceutically acceptable salt (based on total weight of the formulation).

In another embodiment, the stable topical formulation is substantially free of water. The term "substantially free of water" (or "substantially-water-free") in the context of the topical formulation of present invention refers to a topical formulation that contains not more than about 5% w/w of water (based on total weight of the formulation). Preferably, the stable topical formulation of the present invention contains not more than about 3%, or not more than about 2% w/w of water (based on total weight of the formulation). Alternatively, the stable topical formulation of the present invention can be anhydrous (free of water). The water content in the topical formulation of the present invention can be typically determined by a moisture analyzer using the Karl-Fischer (KF) method.

In yet another embodiment, the topical formulation of present invention is stable at accelerated conditions for a period of at least 3 months.

For the topical formulation being referred to as "stable" in the context of present invention, the formulation should contain not more than 9% w/w total impurities (based on total weight of the formulation) formed upon storage at accelerated conditions (i.e., at a temperature of about 40 °C and relative humidity of about 75%) for a period of at least 3 months. As described herein, the topical formulation, before being stored at accelerated conditions, contains less than about 0.5% or 0.2% w/w of total impurities related to voriconazole. Impurity levels above 9% w/w would be unacceptable for a number of reasons, including reduced activity and/or shelf life.

The topical formulation of present invention contains not more than 5.5% w/w impurity A (chemically designated as 2,4-difluoro-2-(1 H)-1,2,4-triazol-I-yl acetophenone) formed upon similar storage for a period of 3 months.

In a specific embodiment of present invention, the stable topical formulation comprising voriconazole or its pharmaceutically acceptable salt is a cream formulation substantially free of water, wherein the cream formulation contains not more than 9% w/w total impurities (based on total weight of the formulation) formed upon storage at a temperature of about 40 °C and relative humidity of about 75% for a period of at least 3 months. Preferably, the cream formulation contains not more than 5.5% w/w impurity A (based on total weight of the formulation) formed upon similar storage for a period of 3 months.

Another specific embodiment of present invention is a stable topical formulation comprising an effective amount of voriconazole or its pharmaceutically acceptable salt (as a first active ingredient), and a corticosteroid (as a second active ingredient), and a pharmaceutical carrier, wherein said topical formulation is substantially free of water. Preferably, the corticosteroid includes clobetasol, halobetasol, fluocinonide, betamethasone, dexamethasone, beclomethasone, alcomethasone, diflorasone, fluticasone, hydrocortisone, mometasone, fluocinolone, desonide, and triamcinolone.

In the context of present invention, for topical use, the effective amount of the corticosteroid ranges from about 0.005% to about 5% w/w, or preferably from about 0.01% to about 3% w/w (based on total weight of the formulation). Preferred weight percentage ranges (based on total weight of the formulation) for various corticosteroids that are contemplated herein include: mometasone (in the range of 0.01% to 1%), betamethasone (in the range of 0.01 to 1 %), fluocinolone (in the range of 0.01 to 1 %), beclomethasone (in the range of 0.01 to 1%), desonide (in the range of 0.01 to 1%), fluticasone (in the range of 0.01 to 1%), hydrocortisone (in the range of 0.01 to 5%), dexamethasone (in the range of 0.01 to 1%), alcomethasone (in the range of 0.01 to 1 %), and diflorasone (in the range of 0.01 to 1%).

More preferably, the stable topical formulation of the present invention includes:
a) voriconazole in the range of about 0.05% to about 2% w/w;
b) mometasone furoate in the range of about 0.01% to about 1% w/w; and
c) a pharmaceutical carrier,
wherein said formulation is substantially free of water.

Yet another embodiment of the present invention relates to a method for treating a local or non-systemic fungal infection in a subject in need thereof, said method comprising applying to an afflicted region of the subject a stable topical formulation comprising an effective amount of voriconazole or its pharmaceutically acceptable salt. Preferably, the subject includes mammal such as human.

The term local or non-systemic fungal infection" in the context of present invention includes topical dermatophytic infections of skin, nail, hair and scalp, and those of oral/nasal/vaginal/rectal mucosa caused by *Tinea, Epidermophyton, Trichophyton* and *Microsporum* species. Fungal infections which may be treated by voriconazole have been extensively described in the literature, including EP 440372, and include topical infections caused by, inter alia, *Candida* spp., *Tinea* spp. *Trichophyton* spp., *Microsporum* spp. or *Epidermophyton floccosum;* mucosal infections caused by *Candida* spp.; systemic infections caused by, inter alia, *Candida* spp., *Cryptococcus neoformans, Aspergillus* spp., *Fusarium* spp., *Scedosporium* spp., *Coccidioides immitis, Paracoccidioides brasiliensis, Histoplasma* spp. or *Blastomyces dermatiditis.*

As used herein, the terms "treating" or "treatment" of a state, disorder or condition mean: (1) inhibiting the state, disorder or condition, i.e., arresting or reducing the development of the disease or at least one clinical or subclinical symptom thereof, or (2) relieving the disease, i.e., causing regression of the state, disorder or condition or at least one of its clinical or subclinical symptoms. The term "treating" or "treatment" as used herein also covers the prophylaxis, mitigation, prevention, amelioration, or suppression of a fungal infection in a subject.

As used herein, the term "subject" includes human and other animals, such as domestic animals (e.g., household pets including cats and dogs) and non-domestic animals (such as wildlife). Preferably, the subject is a human.

The term "active ingredient" (used interchangeably with "active" or "active substance") used herein includes voriconazole or its pharmaceutically acceptable salt.

By "salt" or "pharmaceutically acceptable salt", it is meant those salts and esters which are, within the scope of sound medical judgment, suitable for use in contact with the tissues of humans and lower animals without undue toxicity, irritation, and allergic response, commensurate with a reasonable benefit to risk ratio, and effective for their intended use. Representative acid additions salts include the hydrochloride, hydrobromide, sulphate, and bisulphate. Representative alkali or alkaline earth metal salts include the sodium, calcium, potassium and magnesium salts.

The term "pharmaceutically acceptable" as used in connection with components includes those components approved by a governmental regulatory agency or listed in the U.S. Pharmacopeia or other generally recognized pharmacopeia for use in mammals, such as humans.

One embodiment of the present invention is a stable topical formulation comprising an effective amount of voriconazole or its pharmaceutically acceptable salt and a pharmaceutical carrier, wherein said topical formulation is substantially free of water. Preferably, the stable topical formulation is in the form of a cream or an ointment.

Creams, as well known in the art, are viscous liquids or semisolid emulsions, either oil-in-water or water-in-oil. The oil-in-water cream bases are water-washable, and contain an oil phase, an emulsifier, and an aqueous phase. The oil phase, also called the "internal" phase, is generally comprised of petrolatum and a fatty alcohol such as cetyl or stearyl alcohol. The aqueous phase usually, although not necessarily, exceeds the oil phase in volume, and generally contains a humectant. The emulsifier in a cream formulation is generally a nonionic, anionic, cationic, or amphoteric surfactant.

Ointments are semisolid preparations that are typically based on petrolatum or other petroleum derivatives. The specific ointment base to be used, as will be appreciated by those skilled in the art, is one that will provide for optimum drug delivery, and, preferably, will provide for other desired characteristics as well, e.g., emolliency. As with other carriers or vehicles, an ointment base should be inert, stable, nonirritating and nonsensitizing. As explained in Remington: The Science and Practice of Pharmacy, 19th Ed. (Easton, Pa.: Mack Publishing Co., 1995), at pages 1399-1404, ointment bases may be grouped in four classes: oleaginous bases; emulsifiable -bases; emulsion bases; and water-soluble bases. Oleaginous ointment bases include, for example, vegetable oils, fats obtained from animals, and semisolid hydrocarbons obtained from petroleum. Emulsifiable ointment bases, also known as absorbent ointment bases, contain little or no water and include, for example, hydroxystearin sulfate, anhydrous lanolin and hydrophilic petrolatum. Emulsion ointment bases are either water-in-oil (W/O) emulsions or oil-in-water (O/W) emulsions, and include, for example, cetyl alcohol, glyceryl monostearate, lanolin, and stearic acid. Preferred water-soluble ointment bases are prepared from polyethylene glycols of varying molecular weight; again, see Remington: The Science and Practice of Pharmacy 19th Ed. (Easton, Pa.: Mack Publishing Co., 1995) for further information.

The topical formulation of the present invention contains a pharmaceutical carrier (synonymously, "pharmaceutically acceptable excipient"). Suitable pharmaceutical carriers include, topical carriers, such as, polymers, chelating agents, gelling agents, viscosifying agents, diluents, disintegrants, binders, lubricants, preservatives, surfactants, solvents, emulsifiers, emollients, humectants, buffering agents, chelating agents, and mixtures thereof. Examples of these excipients are described in, for example, Howard C. Ansel et. al., Pharmaceutical Dosage Forms and Drug Delivery Systems, (7th Ed. 1999); Alfonso R. Gennaro et al., Remington: The Science and Practice of Pharmacy, (20th Ed. 2000); and A. Kibbe, Handbook of Pharmaceutical Excipients, (3rd Ed. 2000).

Suitable gelling agents and viscosifying agents include, by way of example, carbomers (CARBOPOL), modified cellulose derivatives, naturally-occurring, synthetic or semi-synthetic gums such as xanthan gum, acacia and tragacanth, sodium alginate, gelatin, modified starches, cellulosic polymers such as hydroxypropyl cellulose, hydroxyethyl cellulose, hydroxypropyl methylcellulose, hydroxypropyl methylcellulose phthalate, and methyl cellulose; co-polymers such as those formed between maleic anhydride and methyl vinyl ether, colloidal silica and methacrylate derivatives, polyethylene oxides, polyoxyethylene-polyoxypropylene copolymers, polyvinyl alcohol and the like and mixtures thereof.

Examples of solvents include water, tetrahydrofuran, isopropyl alcohol, propylene glycol, liquid petrolatum, ether, petroleum ether, alcohols (e.g., methanol, ethanol and higher alcohols), aromatics (e.g., benzene and toluene), alkanes (e.g., pentane, hexane and heptane), ketones (e.g., acetone and methyl ethyl ketone), chlorinated hydrocarbons (e.g., chloroform, carbon tetrachloride, methylene chloride and ethylene dichloride), acetates (e.g., ethyl acetate), oils (e.g., isopropyl myristate, diisopropyl adipate and mineral oil) and mixtures thereof.

Examples of emulsifiers include methyl glucose sesquistearate, PEG-20 methyl glucoside sesquistearate, Steareth-21, polyethylene glycol 20 sorbitan monostearate, polyethylene glycol 60 sorbitan monostearate, polyethylene glycol 80 sorbitan monostearate, Steareth-20, Ceteth-20, PEG-100 stearate, sodium stearoyl sarcosinate, hydrogenated lecithin, sodium cocoylglyceryl sulfate, sodium stearyl sulfate, sodium stearoyl lactylate, PEG-20 glyceryl monostearate, sucrose monostearate, sucrose polystearates, polyglyceryl 10 stearate, polyglcyeryl 10 myristate, steareth 10, DEA oleth 3 phosphate, DEA oleth 10 phosphate, PPG-5 Ceteth 10 phosphate sodium salt, PPG-5 Ceteth 10 phosphate potassium salt, steareth-2, PEG-5 soya sterol oil, PEG-10 soya sterol oil, diethanolamine cetyl phosphate, sorbitan monostearate, diethylenglycol monostearate, glyceryl monostearate, and mixtures thereof.

Examples of emollients include caprylic/capric triglycerides, castor oil, ceteareth-20, ceteareth-30, cetearyl alcohol, ceteth 20, cetostearyl alcohol, cetyl alcohol, cetyl stearyl alcohol, cocoa butter, diisopropyl adipate, glycerin, glyceryl monooleate, glyceryl monostearate, glyceryl stearate, isopropyl myristate, isopropyl palmitate, lanolin, lanolin alcohol, hydrogenated lanolin, liquid paraffins, linoleic acid, mineral oil, oleic acid, white petrolatum, polyethylene glycol, polyoxyethylene glycol fatty alcohol ethers, polyoxypropylene 15-stearyl ether, propylene glycol stearate, squalane, steareth-2 or -100, stearic acid, stearyl alcohol, urea and mixtures thereof.

Examples of film-forming polymers which may be used in the nail lacquer compositions of this invention, include, for example, polyvinyl acetate, mixed polymers (or copolymers) of vinyl acetate with acrylic or methacrylic acid, copolymers of (meth)acrylic acid and (meth)acrylate esters, copolymers of (meth)acrylic acid esters with amino group and/or quaternary ammonium group-containing co-monomers, and the like. These polymers may be used alone or in mixtures with each other or with other film-forming polymers that will not impair the objectives of this invention.

Examples of plasticizers include 1,2,3-propanetriol triacetate (triacetin), dibutyl phthalate, dioctyl phthalate, dibutoxy ethyl phthalate, diamyl phthalate, sucrose acetate isobutyrate, butyl acetyl ricinoleate, butyl stearate, triethyl citrate, dibutyl tartrate, polyethylene glycol, dipropylene glycol, polypropylene glycols, propylene glycol, glycol fatty acid esters, such as, propylene glycol dipelargonate, and mixtures thereof.

Examples of nail permeation enhancers include 2-n-heptyl-1, 3-dioxolane, 2-n-nonyl-1,3-dioxolane, 2-n-undecyl-1,3-dioxolane, 2-n-nonyl-1,3-dioxane, 2-n-undecyl-1,3-dioxane, 2-n-heptylaldehyde-acetal, 2-n-octyl-aldehyde-acetals, e.g., 2-n-octyl-aldehyde-dimethylacetal; 2-n-nonylaldehyde-acetals, 2-n-decylaldehyde-acetals, 3,7-dimethyl-2,6-octadienal (citral) acetals, citronal acetals, 2-n-nonyl-1,3-dioxolane (2-NND), and decanal dimethyl or diethyl acetals, and mixtures thereof, and also includes surfactants.

Examples of humectants include propylene glycol, glycerin, butylene glycol, sorbitol, triacetin and mixtures thereof.

Examples of diluents include lactose, sugar, starches, modified starches, mannitol, sorbitol, inorganic salts, cellulose derivatives (e.g. microcrystalline cellulose, cellulose), calcium sulfate, xylitol, lactitol and the like and mixtures thereof.

Examples of disintegrants include croscarmellose sodium, crospovidone, polyvinylpyrrolidone, sodium starch glycolate, corn starch, microcrystalline cellulose, hydroxypropyl methylcellulose, hydroxypropyl cellulose and the like and mixtures thereof.

Examples of binders include polyvinylpyrrolidone/ povidone, lactose, starches, modified starches, sugars, gum acacia, gum tragacanth, guar gum, pectin, wax binders, microcrystalline cellulose, methylcellulose, carboxymethyl cellulose, hydroxypropyl methylcellulose, hydroxyethyl cellulose, hydroxypropyl cellulose, copolyvidone, gelatin, sodium alginate and the like and mixtures thereof.

Examples of lubricants include magnesium stearate, stearic acid, palmitic acid, calcium stearate, talc, colloidal silicon dioxide, carnauba wax, hydrogenated vegetable oils, mineral oil, polyethylene glycols, sodium stearyl fumarate and the like and mixtures thereof.

Suitable buffering agents include, by way of example, sodium hydroxide, potassium hydroxide, ammonium hydroxide and mixtures thereof.

Suitable chelating agents include mild agents, such as, for example, ethylenediaminetetraacetic acid (EDTA), disodium edetate and EDTA derivatives, and mixtures thereof.

Suitable preservatives include, by way of example, phenoxyethanol, parabens (such as methylparaben and propylparaben), propylene glycols, sorbates, urea derivatives (such as diazolindinyl urea), and mixtures thereof.

The present invention also provides a process for preparing a stable topical formulation comprising voriconazole or its pharmaceutically acceptable salt and pharmaceutically acceptable excipients, wherein the formulation is substantially free from water. Generally, the preparation of a cream formulation as disclosed herein comprises the steps of:
(a) co-melting one or more excipients by heating them up to a temperature of about 75 °C to form a molten mass;
(b) obtaining a dispersion of voriconazole or a salt thereof in a solvent;
(c) mixing the dispersion obtained in step (b) in the molten mass under stirring; and
(d) homogenizing the mixture for about 15-30 min and cooling it gradually to ambient condition.

The stable cream formulation of the present invention can also be prepared by dispersing voriconazole or a salt thereof in a molten cream base, and homogenizing the mixture for about 30 min, and subsequently cooling it.

It will be understood that various modifications may be made to the embodiments disclosed herein. Therefore the above description should not be construed as limiting, but merely as preferred embodiments. Other arrangements and methods may be implemented by those skilled in the art without departing from the scope and spirit of this invention.

The following examples are provided to enable one skilled in the art to practice the invention and are merely illustrative of the invention. The examples should not be read as limiting the scope of the invention.

### EXAMPLES

EXAMPLES 1-3: Topical cream formulation comprising voriconazole.

| **Ingredients** | **Composition (% w/w)** | | |
|---|---|---|---|
| | **Example 1** | **Example 2** | **Example 3** |
| White soft paraffin | 71.5 | 71.0 | 70.0 |
| White wax | 5.0 | 5.0 | 5.0 |
| Propylene glycol stearate | 8.0 | 8.0 | 8.0 |
| Stearyl alcohol and Cateareth 20 (Promulgen-G)* | 7.0 | 7.0 | 7.0 |
| Aluminum starch octenyl succinate | 5.0 | 5.0 | 5.0 |
| Hexylene glycol | 3.0 | 3.0 | 3.0 |
| Voriconazole, micronized | 0.5 | 1.0 | 2.0 |

| | | | |
|---|---|---|---|
| * Commercially available from Lubrizol Inc. | | | |

### Manufacturing process:

Voriconazole was dispersed in hexylene glycol under stirring. All other ingredients were mixed, and the voriconazole dispersion was added to this mixture at about 70 °C under stirring, followed by homogenization for about 15 min. The composition was then gradually cooled to about 36°C to obtain a white to off-white cream.

The water content of these formulations was between 0.5% and 0.8% w/w of the total formulation, as determined by KF method.

### Stability data:

The formulations of Examples 1 through 3 were stored at about 40 °C temperature and a relative humidity of about 75% after packing into aluminum tubes with cap. For six months at monthly intervals, the formulations were analyzed for impurities and an assay of voriconazole was performed. The results of this stability study are tabulated below.

| **Storage time** | **Test parameters** | **Example 1** | **Example 2** | **Example 3** |
|---|---|---|---|---|
| Initial | Impurity A* (% w/w) | 0.02 | 0.02 | 0.02 |
| | Total impurities (% w/w) | 0.05 | 0.01 | 0.05 |
| | Assay of voriconazole (% w/w) | 103.4 | 101 | 100.2 |
| 1 month | Impurity A* (% w/w) | 0.34 | 0.24 | 0.16 |
| | Total impurities (% w/w) | 0.6 | 0.47 | 0.3 |
| | Assay of voriconazole (% w/w) | 99.0 | 100.8 | 99.7 |
| 2 months | Impurity A* (% w/w) | 0.9 | 0.69 | 0.32 |
| | Total impurities (% w/w) | 1.45 | 1.06 | 0.51 |
| | Assay of voriconazole (%) | 100.2 | 100.8 | 102.4 |
| 3 months | Impurity A* (% w/w) | 0.78 | 0.62 | 0.32 |
| | Total impurities (%w/w) | 1.40 | 1.13 | 0.63 |
| | Assay of voriconazole (% w/w) | 99.4 | 105.8 | 103.4 |
| 6 months | Impurity A* (% w/w) | 1.89 | 1.41 | 0.68 |
| | Total impurities (% w/w) | 3.42 | 2.45 | 1.39 |
| | Assay of voriconazole (% w/w) | 98.7 | 98.8 | 99.2 |

| | | | | |
|---|---|---|---|---|
| *Chemically, 2,4-difluoro-2-(1H)-1,2,4-triazol-I-yl acetophenone (formed upon hydrolysis of voriconazole) | | | | |

### Stability indicating method for Voriconazole topical formulation:

### Related Substances (By HPLC):

Reagents: Acetonitrile Methanol Triethylamine Ortho phosphoric acid and Purified Water (Milli Q or equivalent)

### Mobile Phase:

Preparation of Mobile Phase A: 0.2% Triethylamine in water. Adjust pH to 3.0 with Orthophosphoric acid.

Preparation of Mobile Phase B: Acetonitrile: Methanol (50:50, v/v) Preparation of diluent: Acetonitrile: Methanol (50:50, v/v)

### Preparation of Test solution:

Weigh accurately about 75.0 mg of Voriconazole sample and transfer it into a 50mL volumetric flask. Add about 20 mL of diluent and sonicate to dissolve. Make up to the mark with diluent and mix. Filter through 0.45 µm nylon membrane filter.

### Preparation of Diluted Standard Solution:

Weigh accurately about 75 mg of voriconazole in-house working standard and transfer it into a 50 mL volumetric flask. Add about 20 mL of diluent and sonicate to dissolve. Make up to the mark with diluent and mix.

Dilute 5.0 mL of this solution to 100 mL with diluent and mix.

Further dilute 1.0 mL of this solution to 100 mL with diluent and mix.

### Preparation of Placebo Solution:

Weigh Placebo equivalent to 75 mg of Voriconazole sample and transfer it into a 50mL volumetric flask. Add about 20 mL of diluent and sonicate to dissolve. Make up to the mark with diluent and mix.

Filter through 0.45 µ nylon membrane filter.

### Chromatographic Conditions:

Column : C18 Phenomenex Gemini, 250 X 4.6 mm, 5µm
Flow Rate : 1.5 ml/minute
Detection : UV 254 nm
Column temperature : 25°C
Injection volume : 20µl
Run time : 65 minutes
Retention Time : About 25 minutes
Gradient Program :

| Time (minute) | % Mobile Phase A | % Mobile Phase B |
|---|---|---|
| 0.01 | 65 | 35 |
| 30 | 55 | 45 |
| 45 | 20 | 80 |
| 50 | 20 | 80 |
| 55 | 60 | 40 |
| 60 | 65 | 35 |
| 65 | 65 | 35 |

### Evaluation of system suitability:

The relative standard deviation (RSD) of six replicate injections is not more than 5.0%.

Following are the limit of detection/limit of quantification (LOD/ LOQ) parameter for Impurity A formed upon hydrolysis of voriconazole:

| Parameter | RRT | LOD | LOQ | RF |
|---|---|---|---|---|
| Impurity A* | 0.24 | 0.001% | 0.002% | 0.54 |

| | | | | |
|---|---|---|---|---|
| * Chemically, 2,4-difluoro-2-(1H)-1,2,4-triazol-I-yl acetophenone | | | | |

### Procedure:

Inject the equal volumes of the diluent, placebo solution, diluted standard solution and then inject sample solution into the chromatograph and record the chromatograms.

EXAMPLE 4, 5 and Comparative Example A: Cream formulations comprising 1 % w/w voriconazole with varying water contents.

| **Ingredients** | **Composition (% w/w)** | | |
|---|---|---|---|
| | **Example 4** | **Example 5** | **Comparative Example A** |
| White soft paraffin | 71.0 | 66.0 | 61.0 |
| White wax | 5.0 | 5.0 | 5.0 |
| Propylene glycol sterate | 8.0 | 8.0 | 8.0 |
| Stearyl alcohol and Cateareth 20 (Promulgen-G)* | 7.0 | 7.0 | 7.0 |
| Aluminum starch octenyl succinate | 5.0 | 5.0 | 5.0 |
| Hexylene glycol | 3.0 | 3.0 | 3.0 |
| Water | 0.0 | 5.0 | 10.0 |
| Voriconazole, micronized | 1.0 | 1.0 | 1.0 |

The manufacturing process was similar to that described for Examples 1-3.

### Stability data:

The formulations of Example 4, 5 and Comparative Example A were stored at about 40 °C temperature and a relative humidity of about 75% after packing into aluminum tubes with cap. The results of a 1 month stability study are tabulated below.

| **Test parameters** | **Example 4** | | **Example 5** | | **Comparative Example A** | |
|---|---|---|---|---|---|---|
| | Initial | 1-Month | Initial | 1-Month | Initial | 1-Month |
| Assay of voriconazole (% w/w) | 106.5 | 103.7 | 105.0 | 100.7 | 106.8 | 102.8 |
| Total impurities (% w/w) | 0.15 | 0.56 | 0.18 | 2.66 | 0.24 | 3.32 |
| Water by KF (%w/w) | - | 0.45 | - | 4.11 | - | 9.88 |

EXAMPLE 6, 7 and Comparative Example B: Cream formulations comprising 2 % w/w voriconazole with varying water contents.

| **Ingredients** | **Composition (% w/w)** | | |
|---|---|---|---|
| | **Example 6** | **Example 7** | **Comparative Example B** |
| White soft paraffin | 70.0 | 65.0 | 60.0 |
| White wax | 5.0 | 5.0 | 5.0 |
| Propylene glycol sterate | 8.0 | 8.0 | 8.0 |
| Stearyl alcohol and Cateareth 20 (Promulgen-G)* | 7.0 | 7.0 | 7.0 |
| Aluminum starch octenyl succinate | 5.0 | 5.0 | 5.0 |
| Hexylene glycol | 3.0 | 3.0 | 3.0 |
| Water | 0.0 | 5.0 | 10.0 |
| Voriconazole, micronized | 2.0 | 2.0 | 2.0 |

The manufacturing process was similar to that described for Examples 1-3.

### Stability data:

The formulations of Example 6, 7 and Comparative Example B were stored at about 40°C temperature and a relative humidity of about 75% after packing into aluminum tubes with cap. The results of a 1 month stability study are tabulated below.

| **Test parameters** | **Example 6** | | **Example 7** | | **Comparative Example B** | |
|---|---|---|---|---|---|---|
| | Initial | 1-Month | Initial | 1-Month | Initial | 1-Month |
| Assay of voriconazole (% w/w) | 107.7 | 103.3 | 107.7 | 104.3 | 107.1 | 101.7 |
| Total impurities (% w/w) | 0.11 | 0.36 | 0.16 | 1.44 | 0.15 | 1.35 |
| Water by KF (%w/w) | - | 0.35 | - | 3.81 | - | 8.53 |

EXAMPLE 8, 9 and Comparative Example C: Cream formulations comprising 0.5% w/w voriconazole with varying water contents.

| **Ingredients** | **Composition (% w/w)** | | |
|---|---|---|---|
| | **Example 8** | **Example 9** | **Comparative Example C** |
| White soft paraffin | 71.5 | 66.5 | 61.5 |
| White wax | 5.0 | 5.0 | 5.0 |
| Propylene glycol sterate | 8.0 | 8.0 | 8.0 |
| Stearyl alcohol and Cateareth 20 (Promulgen-G)* | 7.0 | 7.0 | 7.0 |
| Aluminum starch octenyl succinate | 5.0 | 5.0 | 5.0 |
| Hexylene glycol | 3.0 | 3.0 | 3.0 |
| Water | 0.0 | 5.0 | 10.0 |
| Voriconazole, micronized | 0.5 | 0.5 | 0.5 |

The manufacturing process was similar to that described for Examples 1-3.

EXAMPLE 10: Stability studies of cream formulations comprising different strengths of voriconazole.

Replica batches of cream formulations comprising different strengths of voriconazole and varying amounts of water were prepared as described above.

The cream formulations were stored at about 40°C temperature and a relative humidity of about 75% after packing into aluminum lacquered collapsible tubes with cap. The results of 1, 2 and 3 months stability study are tabulated below.

| **Cream Formulation** | **Impurity A (% w/w)** | | | **Total impurities (% w/w)** | | |
|---|---|---|---|---|---|---|
| | **1 month** | **2 months** | **3 months** | **1 month** | **2 months** | **3 months** |
| **Voriconazole content: 0.5% w/w** | | | | | | |
| Example 8 | - | 0.15 | 1.08 | - | 0.22 | 1.66 |
| Example 9 | - | 0.20 | 5.08 | - | 0.27 | 7.30 |
| Comparative Example C | - | 1.13 | 6.83 | - | 2.23 | 9.25 |

| **Voriconazole content: 1.0% w/w** | | | | | | |
|---|---|---|---|---|---|---|
| Example 4 | 0.32 | 0.39 | 0.58 | 0.52 | 0.65 | 1.47 |
| Example 5 | 1.60 | 2.66 | 3.33 | 2.59 | 3.94 | 5.28 |
| Comparative Example A | 2.00 | - | - | 3.17 | - | - |

| **Voriconazole content: 2.0% w/w** | | | | | | |
|---|---|---|---|---|---|---|
| Example 6 | 0.23 | 0.19 | 0.24 | 0.46 | 0.28 | 0.66 |
| Example 7 | 0.64 | 1.53 | 1.70 | 1.45 | 1.97 | 4.47 |
| Comparative Example B | 0.95 | 1.51 | - | 1.45 | 1.83 | - |

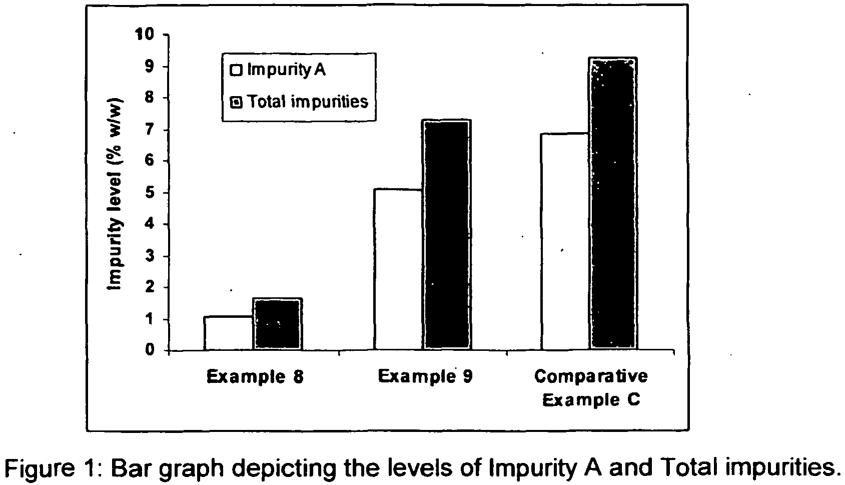

EXAMPLES 11-12: Cream formulations comprising 0.1% and 0.25% w/w voriconazole.

| **Ingredients** | **Composition (% w/w)** | |
|---|---|---|
| | **Example 11** | **Example 12** |
| White soft paraffin | 71.9 | 71.75 |
| White wax | 5.0 | 5.0 |
| Propylene glycol sterate | 8.0 | 8.0 |
| Stearyl alcohol and Cateareth 20 (Promulgen-G)* | 7.0 | 7.0 |
| Aluminum starch octenyl succinate | 5.0 | 5.0 |
| Hexylene glycol | 3.0 | 3.0 |
| Voriconazole, micronized | 0.1 | 0.25 |

The manufacturing process was similar to that described for Examples 1-3.

EXAMPLES 13-15: Shampoo preparation comprising voriconazole 0.1 %, 0.5% and 1% w/w. -

| **Ingredients** | **Composition (% w/w)** | | |
|---|---|---|---|
| | **Example 13** | **Example 14** | **Example 15** |
| Voriconazole | 0.1 | 0.5 | 1.0 |
| Surfadone LP 100* | 10.0 | 10.0 | 10.0 |
| Polyethylene glycol 400 | 62.4 | 62.0 | 61.5 |
| Cocamidopropyl betaine | 2.5 | 2.5 | 2.5 |
| Sodium cocoamphoacetate | 4.0 | 4.0 | 4.0 |
| Sodium lauryl ether sulphate | 7.0 | 7.0 | 7.0 |
| Ammonium lauryl sulphate | 10.0 | 10.0 | 10.0 |
| Hydroxypropyl cellulose | 4.00 | 4.00 | 4.00 |

| | | | |
|---|---|---|---|
| * Chemically, 1-dodecylpyrrolidin-2-one; commercially available from ISP Corp. | | | |

### Manufacturing process:

1. Voriconazole was dissolved in a mixture of Surfadone and polyethylene glycol so as to obtain a clear solution at warm conditions (temperature about 80°C).
2. All other ingredients except hydroxypropyl cellulose were dissolved in the solution of step 1 under stirring. The insoluble particles were filtered out to get a uniform mixture.
3. At about 50°C of the mixture of step 2, hydroxypropyl cellulose was added under stirring so as to obtain thick shampoo preparation.

EXAMPLES 16-18: Nail lacquer preparation comprising voriconazole 0.1 %, 0.5% and 1 %w/w.

| **Ingredients** | **Composition (% w/w)** | | |
|---|---|---|---|
| | **Example 16** | **Example 17** | **Example 18** |
| Ethanol, anhydrous | 56.35 | 55.95 | 55.45 |
| Butyl acetate | 5.0 | 5.0 | 5.0 |
| Ethyl acetate | 15.0 | 15.0 | 15.0 |
| Ammonium Copolymer Methacrylate (type - A) (Eudragit RL 100) | 12.5 | 12.5 | 12.5 |
| Triacetin | 1.05 | 1.05 | 1.05 |
| 2-n-nonyl-1,3-dioxolane/ decanal diethylacetal or decanal dimethylacetal | 10.0 | 10.0 | 10.0 |
| Voriconazole | 0.1 | 0.5 | 1.0 |

### Manufacturing process:

1. Dissolve ammonium copolymer methacrylate in the mixture of ethanol, butyl acetate and ethyl acetate under stirring until solution is obtained. Then add glycerin triacetate (triacetin) by stirring to mix uniformly.
2. Add voriconazole to step 1 and dissolve under stirring to form a solution.
3. Make up the volume with ethanol.

EXAMPLES 19-22: Mouth paint preparations comprising voriconazole 0.5 % and 1 % w/v.

| **Ingredients** | **Composition (% w/v)** | | | |
|---|---|---|---|---|
| | **Example 19** | **Example 20** | **Example 21** | **Example 22** |
| Propylene Glycol | 99.5 | 99.0 | 87.0 | 86.5 |
| Glycerin | - | - | 12.5 | 12.5 |
| Voriconazole | 0.5 | 1.0 | 0.5 | 1.0 |

### Manufacturing process:

1. Voriconazole is dissolved in propylene glycol under stirring until solution is obtained.
2. Wherever applicable, add glycerine to step 1 under stirring.
3. Make up the volume with propylene glycol and filter the final formulation through sieve # 200.

EXAMPLES 23-26: Vaginal tablets formulation comprising voriconazole (100, 200, 300 or 400 mg).

| **Ingredients** | **Composition (% w/w)** | | | |
|---|---|---|---|---|
| | **Example 23** | **Example 24** | **Example 25** | **Example 26** |
| Voriconazole | 11.12 | 20.00 | 28.58 | 36.37 |
| Lactose | 63.73 | 55.00 | 47.14 | 41.50 |
| Pregelatinised starch | 15.00 | 15.15 | 14.29 | 12.00 |
| Croscarmellose sodium | 2.67 | 2.00 | 2.09 | 2.00 |
| Povidone K-30 | 2.00 | 2.50 | 2.48 | 2.50 |
| Purified water | q.s | q.s | q.s | q.s |
| Croscarmellose sodium | 2.00 | 2.00 | 1.71 | 2.00 |
| Magnesium stearate | 1.50 | 1.55 | 1.71 | 1.63 |
| Colloidal silicon dioxide | 1.00 | 0.90 | 1.00 | 1.00 |
| Talc | 1.00 | 0.90 | 1.00 | 1.00 |

### Manufacturing Process:

1. Voriconazole, lactose, pregelatinised starch and croscarmellose sodium are sifted through stainless sieve # 40.
2. All the sifted ingredients of step 1 are blended for 10 minutes.
3. Povidone K-30 is dissolved in water and this binder solution is added to dry mix blend of step 2 to prepare granules.
4. Croscarmellose sodium and magnesium stearate are sifted through sieve # 40, and mixed with granules of step 3 and finally compressed in to tablets so as to get 100, 200, 300 or 400 mg of voriconazole per tablet.

### EXAMPLE 27: Troche formulation comprising voriconazole 50 mg.

| **Step** | **Ingredients** | **Composition (% w/w)** |
|---|---|---|
| I | Voriconazole | 4.88 |
| | Dextrose | 58.53 |
| | Lactose monohydrate | 19.51 |
| II | Povidone K-30 | 5.37 |
| | Isopropyl alcohol | q.s |
| III | Magnesium Stearate | 1.95 |
| | Pregelatinised Starch | 9.76 |

### Manufacturing Process:

1. Voriconazole, dextrose and lactose, are sifted through stainless sieve # 40.
2. All the sifted ingredients of step I are blended for 10 minutes.
3. Povidone K-30 is dissolved in Isopropyl alcohol and this binder solution is added to dry mix blend of step II to prepare granules.
4. Magnesium stearate and pregelatinised starch are sifted through sieve # 40, and mixed with granules of step III and finally compressed in to troches.

EXAMPLES 28 - 29: A topical cream composition comprising voriconazole and mometasone.

| | | **Composition (% w/w)** | |
|---|---|---|---|
| **Step** | **Ingredients** | **Example 28** | **Example 29** |
| I | White Soft Paraffin | 71.0 | 71.5 |
| | White Wax (white Bees wax) | 5.0 | 5.0 |
| | Propylene Glycol Stearate | 8.0 | 8.0 |
| | Stearyl alcohol and Ceteareth-20 (Promulgen-G) | 7.0 | 7.0 |
| | Aluminum Starch Octenylsuccinate | 5.0 | 5.0 |
| II | Hexylene Glycol | 3.0 | 3.0 |
| | Voriconazole | 1.0 | 0.5 |
| | Mometasone | 0.1 | 0.1 |
| | Total | 100 | 100 |

### Manufacturing process:

1. The ingredients of step I are heated at about 70°C.
2. Both the drugs are dispersed in hexylene glycol and added to the bulk of Step I at 70°C under stirring.
3. Homogenize for 15 minutes and cool to room temperature to obtain cream.

EXAMPLE 30: An ointment comprising voriconazole and mometasone.

| **Step** | **Ingredients** | **Composition (% w/w)** |
|---|---|---|
| I | White Soft Paraffin | 77.45 |
| | Liquid Paraffin | 17.0 |
| | White Wax (white Bees wax) | 3.5 |
| | Propylene Glycol Stearate | 1.0 |
| II | Voriconazole | 1.0 |
| | Mometasone | 0.05 |

### Manufacturing process:

1. The ingredients of step I are heated at about 70°C.
2. Both the drugs are dispersed in above phase at 70°C under stirring.
3. Homogenize for 15 minutes and cool to room temperature to obtain an ointment.

## Claims

1. A stable topical formulation in the form of a cream comprising from 0.01% w/w to 5% w/w voriconazole or its pharmaceutically acceptable salt and a pharmaceutical carrier, wherein said formulation contains not more than 5% w/w of water, and not more than 5.5% w/w 2,4-difluoro-2-(1H)-1,2,4-triazol-1-ylacetophenone upon storage at a temperature of about 40°C and a relative humidity of about 75% for a period of 3 months.

2. The topical formulation according to claim 1, wherein the formulation contains not more than 3% w/w of water.

3. The topical formulation according to claim 1, wherein the formulation contains not more than 2% w/w of water.

4. The topical formulation according to any of claims 1 to 3, wherein the formulation comprises from 0.05% w/w to 2% w/w voriconazole or its pharmaceutically acceptable salt.

5. The topical formulation according to any of claims 1 to 3, wherein the formulation comprises from 0.1% w/w to 2% w/w voriconazole or its pharmaceutically acceptable salt.

6. The topical formulation according to any one of claims 1 to 5, wherein the formulation further comprises a corticosteroid that ranges from 0.005% w/w to 5% w/w.

7. The topical formulation according to claim 6, wherein the corticosteroid is selected from the group consisting of clobetasol, halobetasol, fluocinonide, betamethasone, dexamethasone, beclomethasone, alcomethasone, diflorasone, fluticasone, hydrocortisone, mometasone, fluocinolone, desonide, and triamcinolone.

8. The topical formulation according to claim 6, wherein the corticosteroid is mometasone that ranges from 0.01 % w/w to 1% w/w.

9. The topical formulation according to claim 1, wherein the formulation contains 2.0% w/w voriconazole or its pharmaceutically acceptable salt and a pharmaceutical carrier, wherein said formulation contains not more than 2% w/w of water.

10. The topical formulation according to any one of claims 1 to 9, for use in treating a local or non-systemic fungal infection caused by *Tinea, Epidermophyton, Trichophyton and Microsporum* species in a subject in need thereof.

11. A process for the preparation of the topical formulation according to any one of claims 1 to 9, said process comprising:
(a) melting one or more pharmaceutical carrier by heating up to a temperature of about 75°C to form a molten mass;
(b) obtaining a dispersion of voriconazole or its pharmaceutically acceptable salt in a solvent;
(c) mixing the dispersion obtained in step (b) in the molten mass of step (a) under stirring; and
(d) homogenizing the mixture for about 15-30 minutes, and cooling it gradually to ambient condition.

## Patentansprüche

1. Stabile topische Formulierung in Form einer Creme, umfassend von 0,01 Gew% bis 5 Gew% Voriconazol oder sein pharmazeutisch akzeptables Salz und einen pharmazeutischen Träger, wobei die Formulierung nicht mehr als 5 Gew% Wasser und nicht mehr als 5,5 Gew% 2,4-difluoro-2-(1H)-1,2,4-triazol-1-ylacetophenon bei der Lagerung bei einer Temperatur von ungefähr 40 °C und einer relativen Feuchtigkeit von ungefähr 75 % während eines Zeitraums von 3 Monaten enthält.

2. Topische Formulierung nach Anspruch 1, wobei die Formulierung nicht mehr als 3 Gew% Wasser enthält.

3. Topische Formulierung nach Anspruch 1, wobei die Formulierung nicht mehr als 2 Gew% Wasser enthält.

4. Topische Formulierung nach einem der Ansprüche 1 bis 3, wobei die Formulierung von 0,05 Gew% bis 2 Gew% Voriconazol oder sein pharmazeutisch akzeptables Salz enthält.

5. Topische Formulierung nach einem der Ansprüche 1 bis 3, wobei die Formulierung von 0,1 Gew% bis 2 Gew% Voriconazol oder sein pharmazeutisch akzeptables Salz enthält.

6. Topische Formulierung nach einem der Ansprüche 1 bis 5, wobei die Formulierung weiter ein Kortikoid umfasst, das im Bereich von 0,005 Gew% bis 5 Gew% liegt.

7. Topische Formulierung nach Anspruch 6, wobei das Kortikoid ausgewählt ist aus der Gruppe bestehend aus Clobetasol, Halobetasol, Fluocinonid, Betamethason, Dexamethason, Beclomethason, Alcomethason, Diflorason, Fluticason, Hydrocortison, Mometason, Fluocinolon, Desonid und Triamcinolon.

8. Topische Formulierung nach Anspruch 6, wobei das Kortikoid Mometason ist, das im Bereich von 0,01 Gew% bis 1 Gew% liegt.

9. Topische Formulierung nach Anspruch 1, wobei die Formulierung 2,0 Gew% Voriconazol oder sein pharmazeutisch akzeptables Salz und einen pharmazeutischen Träger enthält, wobei die Formulierung nicht mehr als 2 Gew% Wasser enthält.

10. Topische Formulierung nach einem der Ansprüche 1 bis 9 zur Verwendung bei der Behandlung einer lokalen oder nicht-systemischen Pilzinfektion, hervorgerufen von den Spezies *Tinea, Epidemophyton, Trichophyton und Microsporum* in einem Subjekt, das diese benötigt.

11. Verfahren zur Herstellung der topischen Formulierung nach einem der Ansprüche 1 bis 9m wobei das Verfahren Folgendes umfasst:
(a) Schmelzen von einem oder von mehreren pharmazeutischen Trägern durch Erhitzen auf eine Temperatur von ungefähr 75 °C, um eine geschmolzene Masse zu bilden;
(b) Erhalten einer Dispersion von Voriconazol oder seinem pharmazeutisch akzeptablen Salz in einem Lösemittel;
(c) Mischen der Dispersion, die in Schritt (b) erhalten wurde, in die geschmolzene Masse von Schritt (a) unter Rühren; und
(d) Homogenisieren der Mischung während ungefähr 15-30 Minuten und allmähliches Abkühlen dieser auf Umgebungsbedingung.

## Revendications

1. Une formulation topique stable sous la forme d'une crème contenant de 0,01% poids/poids à 5% poids/poids de voriconazole ou de son sel pharmaceutiquement acceptable et un vecteur pharmaceutique, où ladite formulation ne contient pas plus de 5% poids/poids d'eau, et pas plus de 5,5% poids/poids de 2,4-difluoro-2-(1H)-1,2,4-triazol-1-ylacétophénone lors d'un stockage à une température d'environ 40°C et une humidité relative d'environ 75% pendant une période de 3 mois.

2. La formulation topique selon la Revendication 1, où la formulation ne contient pas plus de 3% poids/poids d'eau.

3. La formulation topique selon la Revendication 1, où la formulation ne contient pas plus de 2% poids/poids d'eau.

4. La formulation topique selon l'une quelconque des Revendications 1 à 3, où la formulation contient de 0,05% poids/poids à 2% poids/poids de voriconazole ou de son sel pharmaceutiquement acceptable.

5. La formulation topique selon l'une quelconque des Revendications 1 à 3, où la formulation contient de 0,1 % poids/poids à 2% poids/poids de voriconazole ou de son sel pharmaceutiquement acceptable.

6. La formulation topique selon l'une quelconque des Revendications 1 à 5, où la formulation contient en outre un corticostéroïde qui va de 0,005% poids/poids à 5% poids/poids.

7. La formulation topique selon la Revendication 6, où le corticostéroïde est sélectionné dans le groupe se composant de clobétasol, halobétasol, fluocinonide, bétaméthasone, dexaméthasone, béclométhasone, alcométhasone, diflorasone, fluticasone, hydrocortisone, mométasone, fluocinolone, désonide et triamcinolone.

8. La formulation topique selon la Revendication 6, où le corticostéroïde est mométasone qui va de 0,01 % poids/poids à 1% poids/poids.

9. La formulation topique selon la Revendication 1, où la formulation contient 2,0% poids/poids de voriconazole ou de son sel pharmaceutiquement acceptable et un vecteur pharmaceutique, où ladite formulation ne contient pas plus de 2% poids/poids d'eau.

10. La formulation topique selon l'une quelconque des Revendications 1 à 9, destinée à une utilisation dans le traitement d'une infection fongique locale ou non systémique provoquée par les espèces *Tinea, Epidermophyton, Trichophyton* et *Microsporum* chez un sujet nécessitant un tel traitement.

11. Un processus de préparation de la formulation topique selon l'une quelconque des Revendications 1 à 9, ledit processus comprenant :
(a) la fusion d'un ou de plusieurs vecteurs pharmaceutiques par un chauffage jusqu'à une température d'environ 75°C de façon à former une masse fondue,
(b) l'obtention d'une dispersion de voriconazole ou de son sel pharmaceutiquement acceptable dans un solvant,
(c) le mélange de la dispersion obtenue à l'opération (b) dans la masse fondue de l'opération (a) sous agitation, et
(d) l'homogénéisation du mélange pendant environ 15 à 30 minutes, et son refroidissement graduel vers un état ambiant.
